# FASCICULE DE BREVET EUROPEEN

(11) **EP 1 387 712 B1**
(45) Date de publication et mention de la délivrance du brevet: **02.11.2005**
(21) Numéro de dépôt: 02769443.9
(22) Date de dépôt: 10.05.2002
(51) Int. Cl.: A61N 1/36, A61F 5/01, A61H 1/02, A61H 3/00

(54) **DISPOSITIF DE REEDUCATION ET/OU D'ENTRAINEMENT DES MEMBRES INFERIEURS D'UNE PERSONNE**
THERAPEUTISCHE UND/ODER ÜBUNGSVORRICHTUNG FÜR DIE UNTEREN GLIEDMASSEN EINER PERSON
THERAPEUTIC AND/OR TRAINING DEVICE FOR A PERSON'S LOWER LIMBS

(30) Priorité: 16.05.2001 CH 910012001
(43) Date de publication de la demande: 11.02.2004
(73) Titulaire: Fondation Suisse pour les Cyberthèses, 1844 Villeneuve (CH)
(72) Inventeur: BRODARD, ROLAND, CH-1844 Villeneuve (CH); CLAVEL, Reymond, CH-1041 Oulens-Sous-Echallens (CH)
(74) Mandataire: Ganguillet, Cyril
(86) Numéro de dépôt international: PCT/CH2002/000255
(87) Numéro de publication internationale: WO 2002/092164

(56) Documents cités:
- US-A- 4 653 479
- US-A- 5 255 188
- US-A- 5 954 621
- POPOVIC D ET AL: "HYBRID ASSISTIVE SYSTEM-THE MOTOR NEUROPROSTHESIS" IEEE TRANSACTIONS ON BIOMEDICAL ENGINEERING, IEEE INC. NEW YORK, US, vol. 36, no. 7, 1 juillet 1989 (1989-07-01), pages 729-736, XP000037465 ISSN: 0018-9294

## Description

La présente invention concerne un dispositif de rééducation et/ou d'entraînement des membres inférieurs d'une personne, en particulier d'une personne présentant une atteinte du système nerveux central (paraplégie, hémiplégie).

En l'état actuel de la prise en charge médicale des personnes paralysées méduttaires, on peut constater que sur 100 nouveaux cas, seulement 20% des personnes ont subi une atteinte totale de la moelle épinière et sont, de ce fait, condamnées à une paralysie définitive, mais que 80% ne montrent qu'une atteinte partielle de la moelle épinière.

Compte tenu des possibilités actuelles de la rééducation, il est établi qu'environ 10 à 15% de ce 80% de personnes qui ne sont que partiellement atteintes pourront retrouver une marche autonome de qualité relativement satisfaisante.

Cette récupération s'explique par le fait que le système nerveux central (SNC) et, en particulier, la moelle. épinière démontrent une grande faculté de «plasticité», c'est-à-dire que, par suite d'une réorganisation des circuits nerveux sous-lésionnels de la moelle épinière, des circuits nerveux non atteints, encore sains, peuvent se substituer à des circuits nerveux détruits pour en assurer la fonction.

Cette capacité de substitution par plasticité du système nerveux nécessite un véritable réapprentissage du schéma moteur, qui peut être initié et stimulé par la répétition constante des mouvements usuels que les membres exécutaient avant la paralysie. Cependant, en vue d'atteindre une efficacité maximale de rééducation, ces mouvements devraient être réalisés en respectant le plus étroit mimétisme des mouvements volontaires initiaux, avec une participation active des muscles concernés et en respectant scrupuleusement les charges de résistance qui s'opposaient à ces mouvements.

En effet, pour l'essentiel, cette réorganisation de substitution par plasticité du système nerveux et le réapprentissage du schéma moteur seront déterminés par les informations nerveuses fournies au SNC par le système nerveux proprioceptif et, plus précisément encore, par la boucle fermée de réglage réalisée entre le système nerveux proprioceptif et les nerfs moteurs (motoneurones) des muscles concernés.

Il convient de bien rappeler ici un point essentiel : la contraction de tout muscle responsable d'un mouvement est sous le contrôle du système nerveux proprioceptif dont ledit muscle dépend. Ce système nerveux comprend des propriocepteurs, qui sont des récepteurs, à l'origine d'une fibre nerveuse sensitive, sensibles aux stimulations produites par les mouvements du corps. Ces récepteurs sont situés au voisinage des os, des articulations et des muscles. Le système nerveux proprioceptif (qui représente la sensibilité profonde) forme, avec les muscles qu'il contrôle, un système de réglage en boucle fermée, subtile et précis, qui permet le contrôle des mouvements et de la position du corps.

Il résulte de la paralysie un lourd handicap fonctionnel qui peut être aggravé par tout un cortège de complications : escarres, spasticité, ostéoporose, troubles circulatoires, urinaires, intestinaux et rétractions musculo-tendineuses plus capsulo-ligamentaires.

A une rétraction musculo-teridineuse est associée une atrophie musculaire. Il en résulte que le muscle perd sa force et son endurance. Par conséquent, il perd sa capacité de fournir un travail fonctionnel.

La rétraction capsulo-ligamentaire est également une complication majeure et très fréquente qui peut provoquer une limitation d'amplitude articulaire (ankylose) et des attitudes vicieuses. Avec le temps, elle peut également avoir une répercussion sur le cartilage articulaire.

Il est donc impératif de prévenir ces différentes complications, et en particulier les problèmes musculo-tendino-articulaires, en mobilisant régulièrement le patient dès le début de sa lésion par un programme de rééducation bien établi en fonction de la lésion. Il est également essentiel que, le plus rapidement possible, on verticalise le patient afin qu'il soit dans une position physiologique assurant une régularisation des différents métabolismes et de nombreuses fonctions physiologiques.

Les membres atteints de paralysie sont habituellement soumis à une méthode de mobilisation passive, réalisée par une intervention de force extérieure. Cette force extérieure peut être fournie directement manuellement par un thérapeute ou par un appareillage adéquat.

Pour réaliser en plus un entraînement spécifique en vue de la restauration de la marche, en mettant en jeu une réorganisation de substitution par plasticité de la moelle épinière - telle qu'elle a été décrite plus haut - on peut verticaliser le patient en le soutenant par un harnais sur un tapis roulant, tandis qu'il prend appui avec ses membres supérieurs sur des barres parallèles. Le thérapeute accroupi provoque alors manuellement un mouvement des jambes qui s'apparente à la marche.

C'est un processus aujourd'hui encore usuel, bien qu'il présente nombre d'inconvénients : les mouvements imposés sont lents et imprécis. Le thérapeute est rapidement épuisé par sa position accroupie et le travail fourni. Il en résulte des séances trop courtes avec un nombre réduit de répétitions des mouvements, ce qui diminue considérablement l'efficacité.

Une récente amélioration de ce processus a été réalisée (appareil Lokomat de la société suisse Hocoma AG) par la pure robotisation de ce processus. Une orthèse mécanique fonctionnelle motorisée joue le rôle d'un exosquelette et entraîne un mouvement automatique des membres inférieurs qui reproduit la cinématique de la marche sur un tapis roulant.

L'avantage de cette méthode est qu'elle permet de se passer de l'intervention manuelle d'un thérapeute. Il y a, par conséquent, une amélioration de la rapidité, de la précision et de la répétabilité. Il est également possible de répéter les mouvements à volonté.

Toutefois, toutes les méthodes décrites ci-dessus présentent deux inconvénients majeurs. Premièrement, ce sont toujours des méthodes de mobilisation purement passive des membres. Il en résulte que, faute d'une participation musculaire active, l'efficacité de telles méthodes est limitée et toute l'expérience clinique démontre qu'elles ne suffisent pas à enrayer l'atrophie et l'ankylose des parties paralysées du corps. Deuxièmement, ces méthodes sont purement statiques, le patient restant stationnaire sur un tapis roulant. Par conséquent, il n'y a pas de réapprentissage de la création, puis de la gestion de l'énergie cinétique et de la conservation de la quantité de mouvement, créées par un déplacement du patient et qui constituent la base même de la dynamique de la marche bipède en équilibre dynamique.

Par ailleurs, depuis quelques années, la stimulation électrique fonctionnelle a été proposée pour remplacer la commande physiologique lors de la marche chez les patients ayant certains déficits mineurs. Cette stimulation électrique s'est développée pour stimuler un certain nombre de muscles permettant la position debout (Jaeger et coll., 1990) ou pour améliorer la marche (Carnstam et coll., 1977). Plus récemment, toute une série de travaux ont montré la possibilité, avec un appareillage compliqué et lourd, de restaurer la marche des paraplégiques par stimulation électrique. Cependant, cette rééducation demande une dépense d'énergie considérable et ne s'applique qu'à un certain nombre de patients à lésion médullaire relativement basse. II en résulte que la restauration d'une marche réellement fonctionnelle n'a pas encore été réalisée.

Cet insuccès peut s'expliquer pour une part essentielle par deux inconvénients majeurs communs à toutes les méthodes d'électrostimulation connues à ce jour.

Le premier inconvénient est qu'elles mettent en jeu une électrostimulation classique, la seule utilisée actuellement, dite à réglage en boucle ouverte (Open-Loop Control), caractérisée par le fait que la stimulation est purement et simplement imposée à un muscle, avec une absence de rétrocontrôle (rétroaction) de l'activité ainsi provoquée.

Il faut rappeler que le développement d'un mouvement par stimulation électrique reste délicat : le contrôle de la vitesse et de l'amplitude s'avère sensible et particulièrement difficile lorsque le geste est réalisé avec des charges de résistance additionnelles, ou très rapidement, suite à de fortes impulsions de force qui lui confèrent une forte accélération, comme dans le processus de la marche bipède.

Ce contrôle doit être encore plus précis, répétable et fiable, pour la mobilisation de membres paralysés. En effet, dans un premier temps, la mobilisation doit toujours être douce et progressive, et, par ailleurs, comme il existe en général un déficit sensitif, la personne ne perçoit plus de sensations en provenance du membre paralysé ainsi mobilisé.

Il en résulte que l'utilisation d'une électrostimulation réglée en boucle ouverte ne permet pas un contrôle adéquat des mouvements provoqués. Les mouvements ainsi induits ne sont pas aptes à créer et à gérer l'énergie cinétique et la conservation de la quantité de mouvement, indispensables au bon déroulement d'une marche économique en énergie réellement fonctionnelle. Cela revient à dire que ce type de stimulation n'est pas apte à produire, puis gérer la dynamique du processus de la marche bipède.

En fait, force est de constater que les mouvements ainsi générés s'avèrent tout juste capables d'accomplir, plus ou moins grossièrement, la seule cinématique de la marche. Ce qui revient à dire que ces mouvements sont en quelque sorte suiveurs du déplacement du corps, ce dernier étant généré par une autre voie décrite ci-après.

Le deuxième inconvénient des méthodes d'électrostimulation est pour l'essentiel une conséquence du premier : toutes les méthodes décrites à ce jour nécessitent une importante intervention des membres supérieurs qui prennent appui, soit sur des barres parallèles, soit sur un déambulateur. Le déplacement du corps est dès lors essentiellement provoqué par une avancée manuelle du déambulateur, qui provoque un déplacement vers l'avant du centre de gravité du corps et le place ainsi en position de chute en avant, contrôlée par l'appui des membres supérieurs sur ledit déambulateur. Puis, sous l'action de l'électrostimulation, on provoque un mouvement de suivi d'un membre inférieur puis de l'autre. Ensuite, on redéplace le déambulateur vers l'avant et ainsi de suite.

Il en résulte que la marche ainsi obtenue est constituée d'une suite d'accélérations puis de décélérations (freinages) avec chaque fois un bref arrêt intermédiaire pour déplacer le déambulateur vers l'avant. La conséquence - commune actuellement à toutes les méthodes - est une marche extrêmement lente, au mieux à peine le cinquième de la vitesse normale d'un piéton, nécessitant une énorme dépense d'énergie qui exténue très rapidement le patient. La capacité de déplacement dans de telles conditions n'excède pas quelques dizaines de mètres.

Finalement, ce type de marche orthétique se caractérise en ce qu'elle est statiquement stable sur quatre points d'appui (les deux pieds et les deux mains par l'intermédiaire du déambulateur) et qu'elle ne conserve pas la quantité de mouvement, alors que la marche bipède humaine se caractérise en ce qu'elle permet la maîtrise d'une marche dynamiquement stable sur deux points d'appui avec conservation de la quantité de mouvement.

L'électrostimulation classique, réglée en boucle ouverte, présente un inconvénient supplémentaire quand elle est mise en jeu, en vue d'obtenir un renforcement musculaire. Il a été décrit plus haut que ce type de stimulation ne permet pas un contrôle efficace et adéquat de l'activité dynamique générée par le muscle stimulé, surtout quand ce dernier doit se contracter contre des charges de résistance additionnelles. Ce qui est forcément le cas lors d'un entraînement de renforcement musculaire, en raison du "principe de surcharge" ou "principe d'exercice à résistance progressive".

En effet, il est bien connu que le développement de la force et de l'endurance musculaires - ou musculation -, c'est-à-dire le développement de la capacité maximale de travail, dépend d'un principe appelé "principe de surcharge". D'après ce principe, la force et l'endurance d'un muscle ne se développent que si ce dernier est utilisé pendant un certain temps, au maximum de sa puissance et de son endurance contre une résistance adaptée. Le principe de surcharge implique que la résistance, à laquelle s'oppose le muscle, doit être progressivement augmentée, à mesure que le muscle acquiert de la force et de l'endurance. C'est pour cette raison que l'appellation originelle "principe de surcharge" est maintenant passée sous l'appellation "principe d'exercice à résistance progressive".

Pour des raisons évidentes d'efficacité, de précision et de sécurité, ce type de stimulation réglée en boucle ouverte devrait se confiner essentiellement à l'obtention de contractions isométriques, également dites statiques, car la contraction isométrique se caractérise par une absence de raccourcissement du muscle dont les extrémités restent fixes pendant une contraction.

Si l'on se réfère aux principes de la physique, la conséquence de cette absence de raccourcissement et de mouvement des extrémités du muscle est qu'aucun travail mécanique n'est fourni. Néanmoins, l'activité isométrique consomme de l'énergie qui est dissipée sous forme de chaleur.

Il ressort de la littérature spécialisée que de telles contractions isométriques développent la force isométrique uniquement, et cela encore seulement à un angle articulaire donné et seulement à cet angle.

Or, en règle générale, dans un but de rééducation fonctionnelle, on recherche à augmenter la force et l'endurance d'un muscle sur toute la plage d'angle articulaire physiologique, normalement opérationnelle, qu'il peut engendrer, c'est-à-dire à augmenter sa capacité de fournir un travail mécanique maximum. Il est par conséquent évident que l'électrostimulation réglée en boucle ouverte est inapte à satisfaire ces exigences.

Généralement, quand une personne est atteinte d'une lésion médullaire qui entraîne une paralysie, elle subit, dans un tout premier temps, un choc spinal qui nécessite des soins aigus et une phase initiale au lit. Par la suite, quand on peut entreprendre une première mobilisation précoce de rééducation, qui doit toujours être douce et progressive à ce stade, le patient montre déjà une atrophie musculaire prononcée, consécutive à cette période d'immobilisation.

Par ailleurs, la grande majorité de la population des paraplégiques se trouve actuellement dans une phase très postérieure à la phase initiale au lit et présente de ce fait une atrophie musculaire plus prononcée et fréquemment accompagnée d'ankylose articulaire.

Dans un premier temps, la faiblesse musculaire due à l'atrophie d'immobilisation et les éventuelles restrictions de la-mobilité articulaire des segments du membre dues à l'ankylose ne permettent pas de supporter le poids du corps en position verticale en vue de sa mobilisation. Par conséquent, l'entraînement du renforcement musculaire et de la mobilité articulaire devrait pouvoir être exécuté dans une position favorable à la mobilité totale de chacune des articulations concernées, soit, pour le membre inférieur, de la cheville, du genou et de la hanche. Pour ce faire, une position couchée sur le dos (décubitus dorsal) serait optimale.

Dès lors, il conviendrait d'une part d'entraîner la mobilité articulaire pour renforcer le système ligamentaire et capsulaire, ainsi que pour éliminer très progressivement les éventuelles restrictions angulaires et pour recouvrer, dans toute la mesure du possible, une mobilité complète de l'articulation sur toute son étendue angulaire physiologique. D'autre part, il conviendrait, en parallèle, de procéder à une musculation adéquate, conforme au "principe d'exercice à résistance progressive (principe de surcharge)" et cela sur toute l'étendue angulaire articulaire disponible. Cette musculation devrait pouvoir être exécutée aussi bien dans des mouvements d'extension que de flexion articulaire des différents segments de membre concernés, cela pour éviter de créer une quelconque dysbalance musculaire. Bien entendu, la musculation de muscles paralysés ne peut être réalisée qu'au moyen d'une électrostimulation adéquate.

Au début, ces exercices devraient être réalisés de manière extrêmement douce, précise et progressive, tout particulièrement en ce qui concerne la mobilisation articulaire, pour éviter tout dégât au système musculo-tendino-articulaire et, en particulier, le risque d'ostéome (de type POA) dû à une mobilisation forcée.

Rappelons que la paraostéoarthropathie (POA) consiste en une ossification ectopique qui se développe aux alentours des grosses articulations sous-lésionnelles lors de certaines affections du système nerveux central (paraplégie, hémiplégie, traumatisme crânien,...). Elle représente une complication qui peut être redoutable et redoutée si elle entrave la fonction.

Ainsi, pour toutes les raisons évoquées ci-dessus, les mouvements induits devraient être impérativement et rigoureusement contrôlés en force, vitesse et amplitude articulaire. Comme on l'a vu plus haut, l'électrostimulation classique, réglée en boucle ouverte ne peut pas satisfaire à ces exigences.

L'objectif essentiel de l'entraînement décrit ci-dessus est de permettre au patient de recouvrer, puis d'entretenir une capacité de travail fonctionnel (en quelque sorte un niveau de "fitness") des membres inférieurs qui, dans toute la mesure du possible, soit capable de soutenir le poids du corps en position verticale, ou mieux encore, soit capable de le faire passer d'une position accroupie à la position verticale et réciproquement. A ce stade seulement, le patient sera véritablement apte à pouvoir entreprendre, avec un bénéfice optimal, un entraînement spécifique du processus de la marche bipède.

Il a été décrit précédemment que, dans le cadre d'un entraînement efficace de la marche, la capacité de substitution par plasticité du système nerveux joue un rôle essentiel et que cette capacité est initiée et stimulée par les informations proprioceptives. A cet effet, ainsi que pour le réapprentissage du schéma moteur indispensable, les informations proprioceptives seront d'autant plus efficaces qu'elles seront proches de celles résultant d'une activité volontaire équivalente (principe de mimétisme).

Très schématiquement, et pour l'essentiel, il faut considérer que la marche bipède s'effectue en équilibre purement dynamique sur deux points d'appui. Ce sont de brèves impulsions de force au tout début d'un pas, délivrées par les muscles extenseurs de la cuisse sur la hanche et par les muscles extenseurs du pied, qui assurent la propulsion du corps vers l'avant. Ces impulsions de force, ou impulsions d'élan, provoquent une accélération vers l'avant de la masse corporelle. Ainsi, cette masse corporelle acquiert une vitesse et, par conséquent, une énergie cinétique et une quantité de mouvement. Ces impulsions créent la dynamique du processus de la marche.

Puis, pendant l'exécution du pas, le centre de masse du corps réalise une trajectoire pseudo-balistique d'allure parabolique. Durant l'exécution de cette trajectoire, une participation musculaire relativement faible assure le contrôle de la chaîne cinématique des segments qui composent le membre inférieur. On peut dire qu'à ce stade, la participation musculaire assure le suivi de la trajectoire et, par conséquent, gère la dynamique du processus de la marche.

L'objectif de cette gestion est d'optimiser les mouvements des membres pour minimiser la diminution d'énergie cinétique à la fin du pas et pour conserver ainsi une quantité de mouvement aussi constante que possible, de telle façon qu'au début du pas suivant, une impulsion de force minimale soit suffisante pour compenser la perte d'énergie cinétique et assurer la conservation de la quantité de mouvement, assurant ainsi une propulsion à une vitesse moyenne constante. C'est précisément ce degré de conservation de l'énergie cinétique à la fin de chaque pas qui détermine la quantité d'énergie nouvelle, qui sera nécessaire pour entretenir la propulsion à vitesse moyenne constante.

Le mécanisme décrit ci-dessus est particulièrement bien mis en évidence dans la course à pied. Cette dernière consiste en une accélération de la vitesse de déroulement de la cinématique du processus de la marche. Dans ce cas, lors d'un pas, suite à une forte impulsion d'élan, le centre de masse corporelle suit une trajectoire balistique parabolique, durant laquelle les deux pieds quittent tout contact avec le sol, jusqu'à la réception du poids du corps sur un seul pied à la fin du pas. Puis les mêmes pied et jambe donnent une nouvelle impulsion de force qui assure le pas suivant.

A ce jour il n'existe pas de dispositif de rééducation des membres inférieurs d'une personne paraplégique, ni d'entraînement à la marche, qui remplit les conditions énoncées ci-dessus, de façon à pouvoir entraîner les membres inférieurs d'une manière physiologique, respectant un étroit mimétisme de l'activité volontaire devenue impossible ou restreinte, consécutivement à une paralysie d'origine centrale. De même, aucun des dispositifs proposés jusqu'ici dans la littérature ne permet d'atteindre les conditions énoncées ci-dessus.

Le brevet US 4,642,769 décrit un système de contrôle des mouvements des membres inférieurs, réalisé en chaîne musculaire ouverte, au moyen d'une électrostimulation des muscles paralysés. Il s'agit de moyens externes non incorporés-à l'articulation et ce dispositif paraît difficilement efficace.

Les brevets US 5,476,441 et US 5,954,621 décrivent des dispositifs qui assurent un contrôle de l'angle d'une articulation au moyen d'un freinage rétrocontrôlé de l'angle articulaire. Il s'agit toutefois d'un seul freinage sans possibilité de motorisation de ladite articulation.

Le brevet US 5,682,327 propose un dispositif qui assure le contrôle universel des moteurs et, par conséquent, des mouvements générés au moyen des appareils bien connus de mobilisation passive continue. Comme leur nom le précise bien, ces appareils provoquent des mouvements articulaires purement passifs. Ce dispositif est spécifiquement destiné au contrôlé des attelles mobiles activées par des moteurs. Toutefois, il ne prévoit pas une utilisation associée et contrôlée d'une quelconque électrostimulation neuromusculaire.

Des dispositifs dits hybrides, qui associent une électrostimulation neuromusculaire à une orthèse sont décrits dans WO 96/36278 et dans l'article de Popovic D. et al. : "Hybrid Assistive System - The Motor Neuroprosthesis" (IEEE Transactions on Biomédical Engineering, IEEE Inc. New York, U.S., vol. 36, no 7, July 1, 1989, pages 729-736). Cependant, ces dispositifs présentent un inconvénient majeur, en ce que la masse propre de l'orthèse n'est pas contrôlée. De ce fait, cette masse interfère par ses effets inertiels et gravitationnels, ainsi que par les forces dues aux frottements fonctionnels avec les mouvements désirés des segments corporels électrostimulés, perturbant ainsi profondément la qualité des mouvements souhaités, surtout lorsque les mouvements doivent être exécutés à haute vitesse, contre des résistances de charge, ou pour l'entraînement à la marche.

Le but de la présente invention est de proposer un dispositif de rééducation des membres inférieurs et d'entraînement à la marche, selon le préambule de la revendication 1, qui soit exempt des inconvénients (défauts) énumérés ci-dessus et qui satisfasse les conditions énoncées plus haut, de façon à assurer un entraînement physiologiquement optimal de rééducation des membres inférieurs, puis d'entraînement à la marche, en respectant le plus proche mimétisme d'un entraînement actif volontaire.

A cet effet, l'invention concerne un dispositif de rééducation et/ou d'entraînement des membres inférieurs d'une personne selon la revendication 1.

La réalisation d'un réglage en boucle fermée efficace des mouvements articulaires nécessite de manière incontournable de pouvoir mesurer avec précision les angles articulaires avec des capteurs de position adéquats, ainsi que les forces qui agissent au niveau des articulations au moyen de capteurs de couples adéquats.

En l'état de l'art, il n'existe pas de capteurs adéquats implantables. Ainsi, la mise en oeuvre des capteurs indispensables nécessite de les incorporer dans un exosquelette, qui constitue une orthèse fonctionnelle. Cette orthèse fonctionnelle a également pour fonction de soutenir et de guider les segments des membres pendant les exercices et c'est également à elle que revient la fonction de réaliser et transmettre aux segments des membres les résistances de charge, indispensables à une musculation efficace.

Par conséquent, l'orthèse doit être construite de manière suffisamment robuste pour remplir efficacement toutes les fonctions exigées. La robustesse de l'orthèse ainsi que tous les éléments qui la composent (capteurs, moteurs, etc.) imposent une masse appréciable à chacun de ses éléments mobiles.

Cependant, et c'est une exigence essentielle, durant son fonctionnement et tout particulièrement à grande vitesse, l'orthèse ne doit en aucun cas interférer par sa masse propre avec les conditions physiologiques des mouvements visés et réalisés par le sujet en exercice.

En d'autres termes, tous les effets inertiels, gravitationnels et de frottements fonctionnels de l'orthèse doivent pouvoir être neutralisés, de telle sorte que la présence physique de l'orthèse puisse se faire oublier (être totalement neutralisée), pour que les mouvements exécutés miment librement et fidèlement les mouvements volontaires correspondants.

Ces exigences sont réalisées grâce au contrôle actif des mouvements articulaires propres de l'orthèse, du fait de la motorisation des articulations de l'orthèse contrôlée par le système de réglage en boucle fermée, réalisé avec des capteurs articulaires de position et de couples, reliés à une unité de commande, soumise à un logiciel adéquat.

De préférence, les rétrocontrôles du dispositif de stimulation et des moteurs seront réalisés par des logiciels de commande du type a priori de façon à assurer un réglage continu en temps réel, ces rétrocontrôles distincts sont gérés de manière coordonnée au moyen d'un logiciel adéquat adaptable par programmation à chacun des différents objectifs visés.

De préférence, également, les logiciels de commande incorporent une modélisation mathématique des caractéristiques et des mouvements du dispositif orthétique ainsi qu'une modélisation mathématique du comportement des muscles.

La description qui suit, donnée à titre d'exemple, se réfère au dessin sur lequel :
- la Fig. 1 illustre de façon schématique un premier mode d'exécution du dispositif selon l'invention;
- les Fig. 2a et 2b montrent de façon schématique les deux positions extrêmes de l'orthèse du dispositif de la Fig. 1;
- les Fig. 3a et 3b sont des vues respectivement latérale et depuis dessus d'un exemple de réalisation de l'orthèse de la Fig. 1;
- la Fig. 4 montre un schéma bloc du système de motorisation articulaire du dispositif de la Fig. 1 et de son rétrocontrôle;
- les Fig. 5a et 5b illustrent de façon schématique, respectivement en vue latérale et depuis dessus, un second mode d'exécution du dispositif selon l'invention, et
- la Fig. 6 montre un schéma bloc du système de motorisation articulaire du dispositif de la Fig. 5 et de son rétrocontrôle.

Une orthèse est dite «hybride» quand elle associe une orthèse mécanique fonctionnelle à une électrostimulation neuromusculaire.

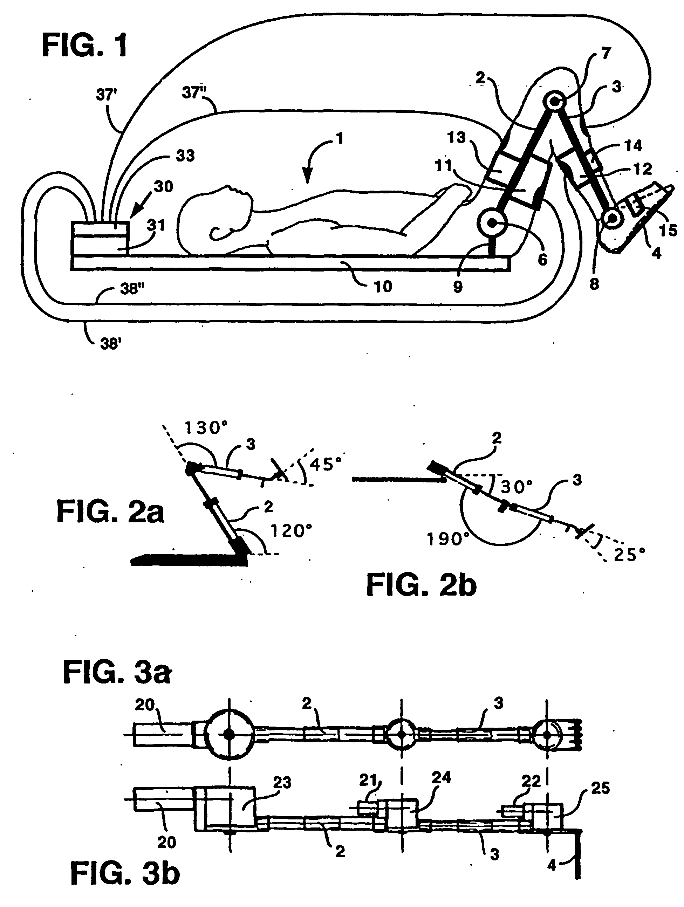

Selon le premier mode d'exécution du dispositif représenté à la Fig. 1, le dispositif comporte une orthèse hybride d'entraînement des membres inférieurs, agencée pour l'utilisation en position couchée sur le dos (décubitus dorsal).

Selon cette forme d'exécution, le dispositif permet l'exercice du mouvement d'une personne accidentée pour rééduquer au mieux les fonctions motrices et articulaires en diminuant les risques d'escarres, en vue de restaurer un usage fonctionnel de ces membres.

Plus précisément, cette orthèse est constituée de deux orthèses identiques, une pour chaque jambe du patient 1, chacune des orthèses étant fixée, par l'intermédiaire d'une articulation 6, et au moyen d'un support réglable 9, à l'extrémité d'un châssis horizontal 10, dûment rembourré et destiné à supporter le dos et le bassin du patient en position couchée sur le dos. Chacune des deux orthèses constitue un système robotique de type sériel, composé de trois segments 2, 3, 4, liés par des articulations 7, 8.

Chacune des orthèses est agencée de façon à réaliser un exosquelette de soutien et de guidage du membre inférieur, assurant ainsi une interface mécanique avec les trois segments corporels qui composent le membre inférieur, à savoir la cuisse, la jambe et le pied.

A cet effet, les parties du membre inférieur (cuisse et jambe) peuvent être liées aux segments correspondants 2, 3 de l'orthèse mécanique au moyen de supports rembourrés en forme de gouttière 11, 12 et de sangles à fermeture du type «velcro» 13, 14 reliés à la structure orthétique.

Comme représenté sur les Fig. 2a, 2b, 3a et 3b, les segments orthétiques 2, 3 de la cuisse et de la jambe sont constitués de tubes télescopiques, dont la longueur peut être adaptée à la morphologie du patient, de telle façon que les articulations orthétiques 6 de la hanche, 7 du genou et 8 de la cheville, coïncident d'un point de vue fonctionnel, avec les articulations physiologiques correspondantes du patient. Le troisième segment orthétique 4 constitue un support plantaire. Le pied est maintenu constamment appuyé contre ce support plantaire au moyen d'une structure souple, qui s'apparente à la structure supérieure d'une chaussure, pouvant être fermée solidement par des languettes souples 15 à fermeture de type «velcro».

L'interface décrite, liant intimement les segments corporels du membre inférieur aux segments orthétiques correspondants, constitue ainsi une unité fonctionnelle : les mouvements du membre et de l'orthèse seront dès lors liés et identiques.

La cinématique de type sériel de chaque orthèse, qui ne comporte qu'une seule chaîne cinématique, est la plus simple qui soit. Les avantages d'un tel système sériel sont assez nombreux, car ce système est très facilement ajustable à la morphologie du patient. On peut le plier facilement et automatiquement. Les trois articulations étant indépendantes, la commande est très simple. Mais surtout, comme on l'a représenté aux Fig. 2a et 2b, la mobilité articulaire autorisée par un tel système est maximale et permet un entraînement optimal de la mobilité articulaire, sur toute son étendue physiologique. En effet, un tel entraînement exige pour chacune des articulations les amplitudes de mouvements, respectivement d'extension et de flexion suivantes :
- Articulation de la hanche : - 5° à 120°
- Articulation du genou : -10° à 130°
- Articulation de la cheville : - 25° à 45°

Toutefois, bien entendu, les orthèses peuvent également être réalisées avec une cinématique de type parallèle.

Comme représenté aux Fig. 3a et 3b, chaque articulation de l'orthèse est actionnée par un servomoteur électrique 20, 21, 22, pouvant avoir un effet moteur ou un effet frein, couplé à un réducteur 23, 24, 25. Chaque moteur est placé à côté d'un des tubes (segments orthétiques) liant les articulations. L'axe du moteur est parallèle au tube adjacent. Il entraîne l'axe perpendiculaire de l'articulation au moyen d'un couple d'engrenages coniques. Ainsi, l'axe du moteur est muni d'un pignon conique qui s'engrène dans une couronne fixée sur l'axe d'entrée du réducteur, l'axe de sortie du réducteur étant rendu solidaire de l'autre tube lié à l'articulation, qu'il peut ainsi entraîner.

Les moteurs permettant de répondre aux exigences de cette application peuvent être des servomoteurs de la marque "Maxon motor". En effet, le poids et les performances en couple de ces moteurs sont excellents. En outre, ces moteurs, bénéficiant d'une construction modulaire, incorporent un codeur digital de position.

Selon une variante, la motorisation de l'articulation s'effectue par un mécanisme à leviers, actionné par une vis de mouvement. Selon cette variante, un moteur est fixé sur un des deux segments d'orthèse rattachés à l'articulation concernée. Ce moteur entraîne la rotation d'une vis sans fin. La rotation de la vis entraîne un écrou monté sur cette vis. Cet écrou, par l'intermédiaire d'une bielle, entraîne le mouvement d'une manivelle. L'axe de rotation de la manivelle correspond à l'axe de rotation de l'articulation et la manivelle est rendue solidaire du second segment d'orthèse rattaché à l'articulation concernée. Il résulte de ce dispositif que selon le sens de rotation du moteur, l'écrou se rapproche du moteur, ce qui provoque une extension de l'articulation. Une rotation inverse du moteur entraîne l'éloignement de l'écrou et provoque une flexion de l'articulation concernée.

Selon une autre variante, l'ensemble peut être agencé de façon que chaque moteur entraîne l'axe perpendiculaire de l'articulation au moyen d'une vis tangente avec un engrenage hélicoïdal gauche. Selon encore une autre variante, les moteurs peuvent être placés perpendiculairement au tube adjacent.

Bien entendu, les servomoteurs électriques peuvent être remplacés par des servomoteurs hydrauliques avec un dispositif de commande hydraulique adéquat.

De même, les codeurs digitaux peuvent être remplacés par des codeurs analogiques.

Des capteurs de forces et de couples sont également intégrés à chaque articulation orthétique.

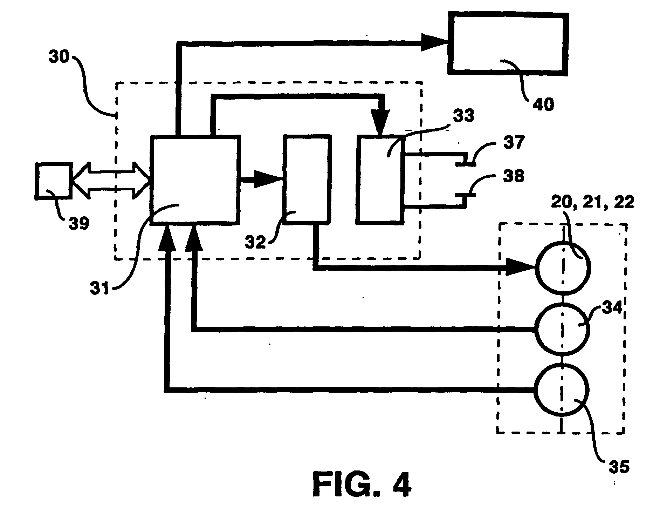

Une unité centrale de commande 30 est intégrée au dispositif. Elle comprend, comme représenté sur le schéma de la Fig. 4, un micro-ordinateur de gestion 31, une alimentation électrique 32 pour les servomoteurs et un stimulateur neuromusculaire électrique 33.

Le stimulateur neuromusculaire électrique 33 est programmable à multicanaux de sortie, par exemple quinze à vingt canaux. Il s'agit d'un générateur d'impulsions électriques, permettant de générer des impulsions de forme quelconque, par exemple de courant rectangulaire d'une durée de l'ordre de 200 à 300 µs chacune. L'amplitude du courant de chaque impulsion peut être réglée entre 0 et 100 mA, tandis que la fréquence de répétition des impulsions peut être réglée entre 5 et 80Hz. Les canaux sont électriquement isolés galvaniquement entre eux et de la terre (sorties flottantes), ceci pour éviter toute interaction électrique intracorporelle entre les canaux en activité. Chaque canal peut être muni d'une paire d'électrodes de surface 37, 38 applicable sur le muscle à stimuler. Seules deux paires d'électrodes 37', 38' et 37", 38" sont représentées sur les Fig: 1 et 5a. Mais bien entendu, d'autres paires d'électrodes peuvent être prévues. De même, les électrodes de surface peuvent être remplacées par un système implanté dans le corps.

Les canaux sont programmables Indépendamment les uns des autres de manière interactive, ceci pour pouvoir provoquer des contractions individuelles, dûment contrôlées en durée et en intensité, de chacun des muscles stimulés, en vue d'une action coordonnée des muscles synergiques et antagonistes d'un mouvement programmé.

Le développement des articulations orthétiques a été dicté par le choix qui a été fait d'y incorporer directement les réducteurs. On a cherché un système relativement compact et applicable, aussi bien au niveau de la hanche, du genou que de la cheville. Le système devant être compact et avoir un facteur de réduction assez élevé, des réducteurs du type "Harmonic Drive" peuvent avantageusement être choisis. Un tel réducteur, d'une conception originale, remplace avantageusement un réducteur classique. Il est compact avec une faible masse. -

La conception adoptée pour les articulations orthétiques permet d'aligner les segments liant les articulations comme représenté aux Fig. 3a et 3b pour que l'ensemble de l'orthèse reste proche de la jambe du patient, dans le but d'améliorer la qualité et la précision de l'unité fonctionnelle réalisée par l'ensemble orthése/jambe.

Le codeur digital de position 34 et les capteurs de forces et de couples 35 intégrés à l'articulation, mobilisée ou freinée par son servomoteur 20, 21 ou 22, transmettent en temps réel leurs informations à l'unité centrale de commande. Le micro-ordinateur de gestion 31 de l'unité de commande 30 interprète ces données, ce qui permet de connaître en temps réel la position angulaire articulaire, l'accélération et la vitesse angulaires de l'articulation, ainsi que les forces et couples qui s'y développent.

Le micro-ordinateur de gestion 31 contient un logiciel dans lequel on a enregistré une modélisation mathématique de l'orthèse, qui tient compte, pour chaque segment de l'orthèse, de sa masse, de son centre de masse et des bras de levier par rapport aux articulations concemées. Les couples aux articulations peuvent également être modélisés. On peut modéliser ainsi toutes les trajectoires libres ou programmables envisageables, comme par exemple les mouvements de passage de la position accroupie à la position verticale, de la position assise à la position verticale, de pédalage, de la marche, etc. Une telle modélisation permet de prévoir tous les mouvements et leurs effets, en particulier les effets inertiels et gravitationnels de l'orthèse. Ceci permet un rétrocontrôle complet en boucle fermée des mouvements de l'orthèse.

En parallèle, la modélisation mathématique du comportement des muscles concernés est enregistrée dans ce logiciel, de façon à permettre le rétrocontrôle en boucle fermée du dispositif de stimulation.

Ces informations constituent ainsi un double rétrocontrôle complet et coordonné de l'activité sous charge de résistance de chaque articulation. Ce rétrocontrôle technique se substitue ainsi à l'absence de rétrocontrôle physiologique, tel qu'il est normalement transmis au système nerveux central (SNC) par le système nerveux proprioceptif. En fait le rétrocontrôle technique mime étroitement le rétrocontrôle physiologique déficient.

Le but essentiel étant de générer des mouvements dûment contrôlés, exécutés contre des charges fixes ou évolutives préprogrammées, la programmation du stimulateur 33 est envisagée de deux manières qui pourront se révéler complémentaires, la seconde pouvant servir à ajuster les paramètres de la première :
1. Analyse des électromyogrammes de mouvements identiques, exécutés en mode volontaire par une personne saine, puis programmation de la séquence musculaire basée sur ces données, en vue de reproduire les mouvements.
2. Programmation purement expérimentale des mouvements par pré-établissement théorique de la séquence musculaire, puis test et ajustement des paramètres en fonction des réactions du patient.

Dans tous les cas, il est prévu que l'opérateur puisse intervenir facilement en cours de stimulation pour modifier la séquence musculaire, c'est-à-dire le début et la fin d'activité d'un canal dans le cycle donné, ainsi que l'ajustage de l'intensité de la contraction.

Les programmes d'entraînement peuvent être spécifiquement établis pour un patient donné, en fonction de sa morphologie, de ses capacités et réactions physiologiques, de ses besoins et des objectifs visés.

Un programme d'entraînement spécifique pour un patient donné peut être préalablement enregistré sur un support de données amovible 39 tel que, par exemple, une disquette ou une carte mémoire. Ce support de données peut être inséré dans l'unité de commande pour que le micro-ordinateur de gestion 31 puisse piloter le stimulateur neuromusculaire 33 et les servomoteurs 20, 21, 22, en vue de réaliser ledit programme d'entraînement.

L'exécution conforme d'un programme d'entraînement est contrôlée par un dispositif dit de «compliance» qui inscrit sur le support de données 39 la valeur des éventuels écarts d'exécution de chacun des paramètres initialement programmés. Cette compliance peut ensuite être interprétée par l'opérateur au moyen d'une relecture du support de données.

La littérature spécialisée met en évidence que si, durant l'exécution de mouvements induits par électrostimulation neuromusculaire, le patient peut être incité à penser à améliorer l'activité ainsi réalisée des membres inférieurs, une telle tâche mentale peut avoir un effet bénéfique marqué qui facilite l'activité en cours.

Par conséquent, on peut adjoindre au dispositif d'entraînement un système de «biofeedback» qui peut, par exemple, consister en un affichage, sur un écran 40 placé devant le patient en exercice, du niveau de performance réalisé, en valeur absolue, et/ou par rapport à un objectif fixé, et/ou encore des progrès accomplis.

L'exécution conforme d'un mouvement programmé, ainsi que d'un enchaînement de mouvements programmés (par exemple la marche) est dûment soumise à un rétrocontrôle, exécuté au moyen des informations transmises au micro-ordinateur de gestion 31 de l'unité centrale de commande par le moyen des capteurs de position 34, ainsi que de force et de couple 35 intégrés à chaque articulation orthétique. Ces informations sont traitées par le micro-ordinateur de gestion 31, dûment programmé à cet effet, et servent dès lors à moduler les paramètres de stimulation qui déterminent la durée et la force d'une contraction, telles que, par exemple, la durée de stimulation, l'intensité du courant et la fréquence de répétition des impulsions pendant la stimulation.

Il en résulte que l'ensemble des informations fournies par les capteurs articulaires (signal de sortie du muscle stimulé), leur traitement au sein du micro-ordinateur au moyen d'un programme adéquat, et la modulation de la stimulation qui en dépend (signal d'entrée du muscle stimulé), constitue un système rétrocontrôlé à réglage en boucle fermée. Un tel système définit une stimulation musculaire électrique rétrocontrôlée à réglage en boucle fermée (Closed-Loop Electrical Muscle Stimulation), capable de générer des mouvements dûment contrôlés et répétables, exécutés contre des charges de résistance fixes ou évolutives préprogrammées, et cela contrairement à une stimulation classique, réglée en boucle ouverte (Open-Loop Control), où il n'y a aucun rétrocontrôle de l'activité musculaire obtenue.

Le dispositif orthétique hybride décrit ci-dessus, combinant et coordonnant les propriétés d'une stimulation musculaire électrique rétrocontrôlée à réglage en boucle fermée et d'une orthèse mécanique fonctionnelle motorisée, également rétrocontrôlée en boucle fermée, constitue un système robotique permettant une extrême polyvalence d'applications, car il peut travailler aussi bien :
- en entraînement, dans les cas où le patient n'a plus aucune force et n'est pas électrostimulé; dans ce cas, seuls les moteurs aux articulations entraînent les mouvements programmés;
- en compensation, quand le patient - électrostimulé ou pas - n'a pas suffisamment de force pour exécuter seul les mouvements et qu'il doit pouvoir être aidé, également avec une compensation de la gravité; dans ce cas, les moteurs aux articulations apportent l'assistance complémentaire nécessaire;
- en freinage, le patient va engendrer - par stimulation et/ou volontairement - un mouvement contre lequel le dispositif va s'opposer en partie ou complètement, au moyen des servomoteurs 20, 21, 22, selon la charge de résistance programmée;
   ainsi, selon la programmation mise en oeuvre :
   si la résistance de charge s'oppose complètement au mouvement, l'entraînement sera isométrique;
   si la résistance de charge s'adapte constamment pour maintenir une vitesse angulaire articulaire constante de mouvement, l'entraînement sera isocinétique;
   si la vitesse angulaire articulaire s'adapte constamment pour maintenir une résistance de charge constante, l'entraînement sera isotonique;
- chaque articulation peut être entraînée individuellement contre une charge de résistance propre programmée. Ainsi, par exemple, il est possible, jambe en extension, d'entraîner au niveau de l'articulation de la cheville la flexion plantaire (c'est-à-dire l'extension du pied) contre une charge de résistance. Un tel entraînement permet de mimer une élévation sur la pointe des pieds contre charge de résistance progressive. Cette dernière pouvant être augmentée graduellement jusqu'à, par exemple, un maximum de 100 kg par jambe. On peut ainsi renforcer l'articulation et les muscles jumeaux, situés dans la loge postérieure de la jambe et qui contribuent pour une part essentielle à l'impulsion de force d'élan dans le processus de la marche.
- les segments corporels, qui constituent le membre inférieur, et leurs articulations peuvent être entraînés de manière coordonnée, conformément à la chaîne cinématique qu'ils constituent, pour produire des mouvements spécifiques :
   par exemple, pour produire des mouvements simultanés d'extension des deux membres inférieurs, suivis de mouvements de flexion contre une charge de résistance, de manière à mimer les mouvements de passage d'une position accroupie à une position-en extension, correspondant à une position verticale du corps, supporté contre la gravité, puis de retour de manière contrôlée à la position accroupie;
   par exemple, pour produire des mouvements alternés de pédalage des deux membres inférieurs contre le freinage résultant d'une charge de résistance programmée;
   par exemple, pour produire par mimétisme, l'entraînement de base des mouvements alternés des deux membres lors de la marche (stepping) contre les résistances correspondantes au niveau des articulations.

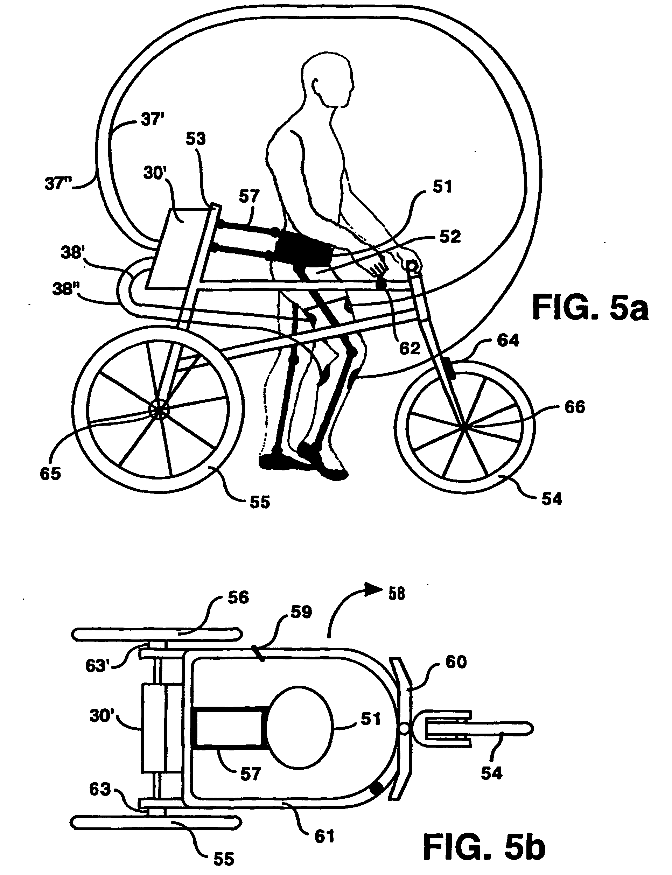

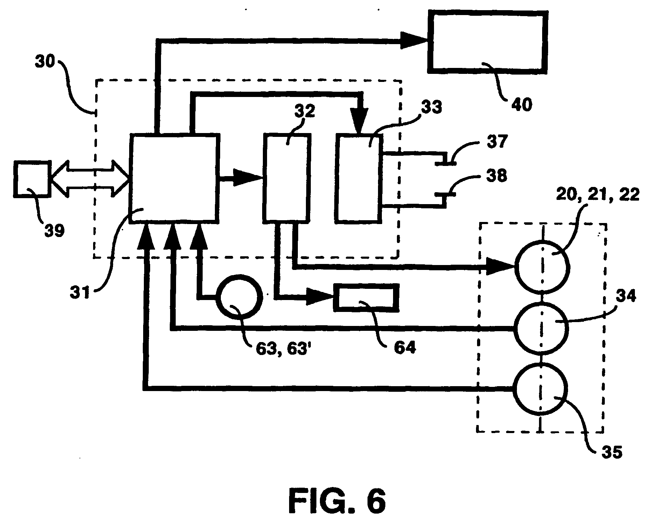

Selon un second mode d'exécution représenté aux Fig. 5a, 5b et 6, le dispositif comprend une orthèse hybride d'entraînement des membres inférieurs semblable à celle de la Fig. 1, mais agencée pour une utilisation en position verticale, en vue de l'entraînement spécifique du processus dynamique de la marche, respectant le déplacement inhérent, lorsque le patient a récupéré un "fitness" (degrés de musculation et de mobilité articulaire) convenable des membres inférieurs, pour entraîner spécifiquement le patient à la marche au moyen d'un étroit mimétisme du processus dynamique de la marche volontaire.

A cet effet, tous les éléments du dispositif selon le premier mode d'exécution, à l'exception du châssis horizontal 10 destiné à supporter le dos et le bassin du patient en position couchée sur le dos, peuvent être réutilisés, les deux orthèses identiques destinées aux deux jambes étant dans ce cas fixées de chaque côté d'une ceinture pelvienne mécanique 51, réglable de façon que les articulations orthétiques de la hanche coïncident d'un point de vue fonctionnel avec les articulations physiologiques correspondantes du patient. La ceinture pelvienne, constituant en quelque sorte un exobassin, est munie d'une sorte de culotte 52 qui constitue un harnais destiné à supporter le tronc du patient.

L'ensemble constitué par la ceinture pelvienne et la culotte harnais contribue également à assurer la stabilité verticale du tronc. Selon les cas, cet ensemble peut encore être complété par deux montants latéraux, réunis par une ceinture thoracique pour améliorer encore la stabilité verticale du tronc.

La ceinture pelvienne 51 est fixée par un dispositif mécanique articulé à un châssis mobile 53, structure légère munie de trois roues 54, 55, 56 pour assurer son déplacement sur le sol dans la direction de la marche. Le dispositif mécanique de liaison de la ceinture pelvienne avec le châssis est conçu de façon qu'il ne permette qu'un déplacement vertical de la ceinture pelvienne, pour lui permettre de suivre le faible déplacement vertical d'aller et retour dû à la trajectoire d'allure parabolique, qui se développe pendant la marche. Par ailleurs, ce dispositif doit être suffisamment rigide pour assurer la stabilité au plan horizontal de la ceinture pelvienne et son suivi de la trajectoire imposée par le châssis lors d'un changement de direction imposé par le patient en déplacement. A titre d'exemple, un tel dispositif de liaison mécanique peut être réalisé au moyen d'un parallélogramme articulé 57 placé dans le plan vertical longitudinal, dont l'un des éléments verticaux est fixé au châssis et l'autre élément vertical du côté opposé est fixé à la ceinture pelvienne au moyen d'une fixation amovible à verrouillage rapide. Le parallélogramme peut être muni d'un dispositif de motorisation; tel que par exemple un servomoteur agissant dans le plan vertical et asservi à des capteurs de force et de couple ainsi que de position. Un tel dispositif est ainsi capable de supporter en tout ou partie le poids du corps pendant son déplacement. Le châssis peut également s'ouvrir sur l'un de ses côtés 58, dont les montants à verrouillage rapide 59 peuvent pivoter vers l'avant pour dégager l'accès à l'intérieur du châssis.

En effet, il est prévu d'appareiller au préalable le patient avec son orthèse, puis de l'introduire à l'intérieur du châssis et de fixer la ceinture pelvienne à son dispositif de liaison avec ledit châssis.

La roue avant 54 est orientable, commandée par un guidon 60 actionné manuellement par le patient, pour effectuer des changements de direction et, en particulier, assurer un aller et retour sur la piste d'entraînement. On peut également utiliser un dispositif du type à roue folle. Dans ce cas, les changements de direction sont imposés par le changement de la direction de la marche du patient, changement de direction provoqué par le dispositif de stimulation. A cet effet, contrairement à ce qui est illustré à la Fig. 5b, la position optimale du patient est à l'axe des roues arrières.- Selon cette variante, le guidon peut bien entendu être conservé de façon à permettre des corrections manuelles de la direction.

Le châssis est doté sur son pourtour d'une balustrade, ou rampe d'appui 61 à portée de main du patient. Cette rampe, ainsi que la ceinture pelvienne et le guidon de direction sont ajustables en hauteur pour pouvoir être adaptés à la morphologie du patient.

L'unité centrale de commande 30' est également fixée audit châssis, de même qu'une commande 62 placée à portée de main du patient, pour démarrer, contrôler la vitesse, puis arrêter l'entraînement à la marche.

Etant donné que, lors d'un changement de direction imposé par le patient, la roue extérieure au virage suit une trajectoire plus longue que la roue intérieure, il en sera de même de la trajectoire plus importante à parcourir par les enjambées extérieures au virage que par les enjambées intérieures au virage du patient. Par conséquent, pour éviter une trop forte contrainte physiologique différentielle sur les membres inférieurs, les deux roues concernées sont munies de capteurs codeurs digitaux 63, 63', dont les informations traitées par l'unité centrale de commande agissent en conséquence sur le réglage en boucle fermée de stimulation, pour moduler de manière adéquate la stimulation musculaire appliquée aux deux jambes.

Bien qu'un tel processus soit quelque peu simpliste et ne remplisse pas toutes les conditions physiologiques d'un changement de virage volontaire, il s'avère néanmoins suffisant dans le cas de l'entraînement décrit ici.

Le châssis mobile peut être muni d'un frein 64, agissant par exemple sur la roue avant. Ce frein est dûment asservi à la vitesse de déplacement du patient et au moyen des codeurs digitaux 63, 63' des roues arrières pour synchroniser parfaitement la vitesse de déplacement du châssis avec la vitesse de déplacement propre du patient due à son activité de marche.

Le châssis mobile peut d'autre part comporter son propre dispositif d'entraînement, constitué par exemple d'un ou plusieurs moteurs ou servomoteurs 65, 66 asservis à l'unité centrale de commande 30 et agissant sur l'une ou plusieurs des roues 54, 55, 56, pour synchroniser parfaitement la vitesse de déplacement dudit châssis mobile avec la vitesse de déplacement propre du patient due à son activité de marche. Une telle disposition est notamment particulièrement utile dans le cas où le châssis mobile est alourdi par la présence de batteries pour son alimentation électrique.

Le dispositif décrit ci-dessus présente l'avantage fondamental qu'il permet de réaliser un entraînement à la marche, absolument conforme à son processus physiologique volontaire dynamique, qu'il permet de mimer parfaitement

Ainsi, une stimulation musculaire conforme au processus connu de la marche, réalisée de manière parfaitement rétrocontrôlée par réglage en boucle fermée (voir schéma de la Fig. 6 analogue à celui de la Fig. 4), peut permettre de donner l'impulsion d'élan, parfaitement dosée, indispensable pour provoquer la propulsion. De ce fait, la masse du corps subit une accélération et acquiert une certaine vitesse de déplacement. Il en résulte la création d'une énergie cinétique et d'une quantité de mouvement. Le dispositif proposé, qui permet un déplacement effectif, suiveur de la marche, autorise par conséquent la gestion de l'énergie cinétique ainsi créée et, en particulier, une conservation de la quantité de mouvement, quasiment identiques à ce qui est réalisé lors d'une marche volontaire équivalente. Comme le système permet une prise en charge partielle ou complète du poids du corps par les membres inférieurs lors de la marche, le travail effectué, la vitesse de déplacement atteinte et, finalement, l'énergie nécessaire consommée sont pratiquement identiques à ceux d'un déplacement volontaire équivalent.

Dans ces conditions, les influx proprioceptifs délivrés au système nerveux central sont très proches de ceux fournis par un entraînement volontaire. Par conséquent, ils peuvent avoir une action optimale de stimulation de la capacité de substitution par plasticité du système nerveux central, en vue de tester puis d'entraîner l'éventuelle restauration d'une marche volontaire satisfaisante d'un point de vue fonctionnel, lorsque la moelle n'est que partiellement atteinte. Pour les mêmes raisons, le réapprentissage du schéma moteur de la marche est optimal.

Selon une variante du second mode d'exécution qui vient d'être décrit, les articulations 6, 7 et 8 sont dépourvues de moteurs ou freins. Mais bien entendu, les capteurs angulaires et les capteurs de force restent nécessaires.

D'autre part, quel que soit le mode d'exécution, les moteurs peuvent ne pas être embarqués sur les orthèses, mais être montés sur tout autre support, par exemple le support 10 de la Fig. 1 ou sur le dispositif de support déplaçable des Fig. 5a et 5b, et reliés mécaniquement à leurs réducteurs correspondants au moyen d'une commande à distance, par exemple à transmission par chaînes et pignons.

Bien entendu, tous les dispositifs décrits ci-dessus peuvent également être utilisés dans le domaine de l'entraînement sportif.

## Revendications

1. Dispositif de rééducation et/ou d'entraînement des membres inférieurs d'une personne, en particulier d'une personne présentant une atteinte du système nerveux central comme la paraplégie et l'hémiplégie, comprenant un dispositif orthétique mécanique (2, 3, 4) agencé pour constituer une interface avec au moins un des membres inférieurs du patient et un dispositif de stimulation neuro-musculaire (33) comportant au moins une paire d'électrodes (37, 38) destinées à agir sur le muscle ou le groupe musculaire concerné dudit membre de la personne, ledit dispositif orthétique comportant au moins une articulation (6, 7, 8) munie d'un capteur angulaire et d'au moins un capteur de force, lesdits capteurs étant couplés au dispositif de commande (31) du dispositif de stimulation, **caractérisé en ce que** ladite articulation du dispositif orthétique est pourvue d'un moteur d'actionnement de l'orthèse commandé par un système de réglage en continu en boucle fermée en temps réel à l'aide desdits capteur angulaire et capteur de force, de façon coordonnée avec des moyens de rétrocontrôle à réglage en continu en boucle fermée en temps réel dudit dispositif de stimulation.

2. Dispositif selon la revendication 1, **caractérisé en ce que** ledit système de réglage du moteur comprend un logiciel de commande du type a priori.

3. Dispositif selon l'une des revendications 1 ou 2, **caractérisé en ce que** lesdits moyens de rétrocontrôle du dispositif de stimulation comprennent un logiciel de commande du type a priori.

4. Dispositif selon la revendication 2 ou la revendication 3, **caractérisé en ce que** le ou lesdits logiciel(s) de commande incorporent une modélisation mathématique des caractéristiques et des mouvements du dispositif orthétique ainsi qu'une modélisation mathématique du comportement des muscles du patient.

5. Dispositif selon l'une des revendications 1 à 4, **caractérisé en ce que** le dispositif orthétique comprend au moins une orthèse comportant au moins trois segments (2, 3, 4) destinés à constituer une interface mécanique avec respectivement la cuisse, la jambe et le pied du patient, les premier et deuxième segments (2, 3) comportant des moyens (11, 13; 12, 14) pour leurs liaisons respectives à la cuisse et à la jambe du patient et le troisième segment (4) étant disposé de façon à constituer un support plantaire et comportant des moyens (15) pour sa fixation au pied du patient, le premier segment étant relié d'une part à l'une de ses extrémités par une première articulation motorisée (6) à un élément agencé pour coopérer avec le corps du patient au niveau de ses hanches et d'autre part à son autre extrémité par une deuxième articulation motorisée (7) à l'une des extrémités du second segment, l'autre extrémité dudit second segment étant renée par une troisième articulation motorisée (8) au troisième segment.

6. Dispositif selon la revendication précédente, **caractérisé en ce qu'**il comporte deux dites orthèses dont chacune est agencée pour constituer une interface avec l'un des membres inférieurs du patient.

7. Dispositif selon l'une des revendications 5 ou 6, **caractérisé en ce que** lesdits premier et deuxième segments sont constitués d'éléments de longueurs réglables, de façon à pouvoir adapter leur longueur à la morphologie du patient

8. Dispositif selon l'une des revendications précédentes, **caractérisé en ce que** chacune des articulations de chacune des orthèses est solidaire d'un servomoteur électrique couplé à un réducteur.

9. Dispositif selon la revendication 8, **caractérisé en ce que** l'axe de chacun des moteurs est parallèle au segment d'orthèse adjacent et **en ce qu'**il entraîne l'axe perpendiculaire de l'articulation au moyen d'un couple d'engrenages coniques.

10. Dispositif selon la revendication 8, **caractérisé en ce que** le moteur est fixé sur un des deux segments d'orthèse rattachés à l'articulation et entraîne en rotation une vis sans fin, sur laquelle est montée un écrou qui, à son tour, entraîne le mouvement d'une manivelle, solidaire du second segment d'orthèse, et dont l'axe de rotation correspond à l'àxe de rotation de l'articuiation.

11. Dispositif selon la revendication 10, **caractérisé en ce que** l'axe de chacun des moteurs est perpendiculaire au segment d'orthèse adjacent.

12. Dispositif selon l'une des revendications 8 à 11, **caractérisé en ce que** chacun des moteurs incorpore un codeur digital de position et **en ce que** des capteurs de force et de couple sont intégrés à chaque articulation.

13. Dispositif selon l'une des revendications 8 à 12, **caractérisé en ce que** les moteurs sont asservis au dispositif de commande (31), et **en ce que** ce dernier comporte des moyens de commande séparée de chacun desdits moteurs.

14. Dispositif selon l'une des revendications précédentes, **caractérisé en ce que** le dispositif orthétique est fixé à un châssis horizontal (10) agencé pour supporter le dos et le bassin du patient pour un entraînement de ses membres inférieurs en position horizontale.

15. Dispositif selon l'une des revendications 1 à 13, **caractérisé en ce que** le dispositif orthétique est fixé à une ceinture pelvienne mécanique (51) agencée pour être placée autour des hanches du patient pour un entraînement des membres inférieurs du patient en position verticale, ladite ceinture pelvienne comportant des organes pour sa fixation à un dispositif de support déplaçable de façon à permettre au patient de se déplacer.

16. Dispositif selon la revendication 15, **caractérisé en ce que** la ceinture pelvienne est fixée au dispositif de support déplaçable par un mécanisme articulé.

17. Dispositif selon la revendication 16, **caractérisé en ce que** ledit mécanisme articulé est muni d'un dispositif de motorisation agissant dans le plan vertical et asservi à des capteurs de force, de couple et de position, de façon à supporter en tout ou partie le poids du corps du patient pendant son déplacement

18. Dispositif selon l'une des revendications 15 à 17, **caractérisé en ce que** ledit dispositif de support est monté sur des roues (54, 55, 56).

19. Dispositif selon l'une des revendications 15 à 18, **caractérisé en ce que** ledit dispositif de support comprend son propre dispositif d'entraînement (65, 66) asservi au dispositif de commande (31).

20. Dispositif selon l'une des revendications 1 à 19, **caractérisé en ce que** le système de commande (31) comprend un micro-ordinateur.

21. Dispositif selon la revendication 20, **caractérisé en ce que** le système de commande (31) comprend des moyens de lecture destinés à coopérer avec des supports de données emovibles de façon à permettre la réalisation de programmes d'entraînement individualisés.

22. Dispositif selon l'une des revendications précédentes, **caractérisé en ce qu'**il comprend des moyens d'affichage (40) destinés en particulier à informer le patient en temps réel de son niveau de performance.

## Claims

1. A device for re-educating and/or training the lower limbs of a person, in particular a person having an impairment of the central nervous system (paraplegia, hemiplegia), comprising a mechanical orthetic device (2, 3, 4) arranged to constitute an interface with at least one of the lower limbs of the patient and a neuromuscular stimulation device (33) comprising at least one pair of electrodes (37, 38) intended to act on the relevant muscle or muscle group of the said limb of the patient, the said orthetic device comprising at least one articulation (6, 7, 8) provided with an angular sensor and at least one force sensor, the said sensors being coupled to the device (31) controlling the stimulation device, **characterised in that** the said articulation of the orthetic device is provided with an actuating motor of the orthesis controlled by a closed-loop continuous control system in real time by means of the said angular sensor and force sensor, in a manner which is coordinated with closed-loop continuously controlled in real time retrocontrol means of said stimulation device.

2. A device according to claim 1, **characterised in that** said control system of the motor comprises a control software of the a priori type.

3. A device according to one of claims 1 or 2, **characterised in that** said retrocontrol means of said stimulation device comprise a control software of the a priori type.

4. A device according to claim 2 or claim 3, **characterised in that** the said control software incorporate a mathematical modelling of the characteristics and movements of the orthetic device and a mathematical modelling of the behaviour of the muscles of the patient.

5. A device according to one of claims 1 to 4, **characterised in that** the orthetic device comprises at least one orthesis comprising at least three segments (2, 3, 4) intended to constitute a mechanical interface with respectively the thigh, the leg and the foot of the patient, the first and second segments (2, 3) comprising means (11, 13; 12, 14) for their respective connections to the thigh and to the leg of the patient and the third segment (4) being disposed so as to constitute a plantar support and comprising means (15) for its fixing to the foot of the patient, the first segment being connected firstly at one of its ends by a first motorised articulation (6) to an element arranged so as to cooperate with the body of the patient at his hips and on the other hand at its other end by a second motorised articulation (7) to one of the ends of the second segment, the other end of the said second segment being connected by a third motorised articulation (8) to the third segment.

6. A device according to the preceding claim, **characterised in that** it comprises two said ortheses, each of which is arranged so as to constitute an interface with one of the lower limbs of the patient.

7. A device according to one of claims 5 or 6, **characterised in that** the said first and second segments consist of elements with adjustable lengths, so as to be able to adapt their length to the morphology of the patient.

8. A device according to one of the preceding claims, **characterised in that** each of the articulations of each of the ortheses is secured to an electric servomotor coupled to a gearbox.

9. A device according to claim 8, **characterised in that** the shaft of each of the motors is parallel to the adjacent orthesis segment and **in that** it drives the perpendicular shaft of the articulation by means of a pair of bevel gears.

10. A device according to claim 8, **characterised in that** the motor is fixed to one of the two orthesis segments attached to the articulation and rotationally drives a worm, on which a nut is mounted which in its turn drives the movement of a crank, fixed to the second orthesis segment, and whose rotation axis corresponds to the rotation axis of the articulation.

11. A device according to claim 10, **characterised in that** the shaft of each of the motors is perpendicular to the adjacent orthesis segment.

12. A device according to one of claims 8 to 11, **characterised in that** each of the motors incorporates a digital position coder and **in that** force and torque sensors are integrated in each articulation.

13. A device according to one of claims 8 to 12, **characterised in that** the motors are slaved to the control device (31) and **in that** the latter comprises separate control means for each of the said motors.

14. A device according to one of the preceding claims, **characterised in that** the orthetic device is fixed to a horizontal frame (10) arranged so as to support the back and pelvis of the patient for driving his lower limbs in the horizontal position.

15. A device according to one of claims 1 to 13, **characterised in that** the orthetic device is fixed to a mechanical pelvic belt (51) arranged so as to be placed around the hips of the patient for driving the lower limbs of the patient in the vertical position, the said pelvic belt comprising members for fixing it to a movable support device so as to enable the patient to move.

16. A device according to claim 15, **characterised in that** the pelvic belt is fixed to the movable support by an articulated mechanism.

17. A device according to claim 16, **characterised in that** the said articulated mechanism is provided with a motorisation device acting in the vertical plane and slaved to force, torque and position sensors, so as to wholly or partly support the weight of the body of the patient during his movement.

18. A device according to one of claims 15 to 17, **characterised in that** the said support device is mounted on wheels (54, 55, 56).

19. A device according to one of claims 15 to 18, **characterised in that** the said device comprises its own drive device (65, 66) slaved to the control device (31).

20. A device according to one of claims 1 to 19, **characterised in that** the control system (31) comprises a microcomputer.

21. A device according to claim 20, **characterised in that** the control system (31) comprises reading means intended to cooperate with removable data media so as to enable individualised training programs to be produced.

22. A device according to one of the preceding claims, **characterised in that** it comprises display means (40) intended in particular to inform the patient in real time of his performance level.

## Patentansprüche

1. Vorrichtung zur Rehabilitation und/oder zum Training der unteren Gliedmaßen einer Person, insbesondere einer Person, die eine Schädigung des Zentralnervensystems, wie z.B. Querschnittlähmung und Halbseitenlähmung, aufweist, umfassend eine mechanische orthopädische Vorrichtung (2, 3, 4), die so ausgebildet ist, dass sie eine Schnittstelle mit mindestens einer der unteren Gliedmaßen des Patienten und einer Vorrichtung zur Nerven- und Muskelstimulation (33) bildet, die mindestens ein Paar Elektroden (37, 38) umfasst, die dazu bestimmt sind, auf den betreffenden Muskel oder die betreffende Muskelgruppe der Gliedmaße der Person einzuwirken, wobei die orthopädische Vorrichtung mindestens ein Gelenk (6, 7, 8) umfasst, das mit einem Winkelsensor und mindestens einem Kraftsensor versehen ist, wobei die Sensoren an die Steuervorrichtung (31) der Stimulationsvorrichtung gekoppelt sind, **dadurch gekennzeichnet, dass** das Gelenk der orthopädischen Vorrichtung mit einem Motor zum Antrieb der Prothese versehen ist, der mit Hilfe des Winkelsensors und des Kraftsensors durch ein System zur kontinuierlichen Regelung in geschlossenem Regelkreis und in Echtzeit gesteuert wird, und zwar auf koordinierte Weise mit den kontinuierlich, in geschlossenem Regelkreis und in Echtzeit geregelten Rückkopplungsmitteln der Stimulationsvorrichtung.

2. Vorrichtung nach Anspruch 1, **dadurch gekennzeichnet, dass** das System zur Regelung des Motors eine Steuersoftware vom Typ a priori umfasst.

3. Vorrichtung nach Anspruch 1 oder 2, **dadurch gekennzeichnet, dass** die Rückkopplungsmittel der Stimulationsvorrichtung eine Steuersoftware vom Typ a priori umfassen.

4. Vorrichtung nach Anspruch 2 oder Anspruch 3, **dadurch gekennzeichnet, dass** das oder die Steuersoftwareprogramm(e) eine mathematische Modellisierung der Merkmale und Bewegungen der orthopädischen Vorrichtung sowie eine mathematische Modellisierung des Verhaltens der Muskeln des Patienten umfassen.

5. Vorrichtung nach einem der Ansprüche 1 bis 4, **dadurch gekennzeichnet, dass** die orthopädische Vorrichtung mindestens eine Prothese umfasst, die mindestens drei Segmente (2, 3, 4) aufweist, die dazu bestimmt sind, eine mechanische Schnittstelle mit dem Oberschenkel, dem Unterschenkel bzw. dem Fuß des Patienten zu bilden, wobei das erste und zweite Segment (2, 3) Mittel (11, 13; 12, 14) zu seiner Verbindungen mit dem Oberschenkel bzw. dem Unterschenkel des Patienten umfassen und das dritte Segment (4) so angeordnet ist, dass es eine Fußsohlenstütze bildet, und Mittel (15) zu seiner Befestigung am Fuß des Patienten umfasst, wobei das erste Segment wie folgt verbunden ist: einerseits an einem seiner Enden durch ein erstes motorisiertes Gelenk (6) mit einem Element, das so ausgebildet ist, dass es mit dem Körper des Patienten im Bereich seiner Hüften zusammenarbeitet, und andererseits an seinem anderen Ende durch ein zweites motorisiertes Gelenk (7) mit einem der Enden des zweiten Segments, wobei das andere Ende des zweiten Segments durch ein drittes motorisiertes Gelenk (8) mit dem dritten Segment verbunden ist.

6. Vorrichtung nach dem vorhergehenden Anspruch, **dadurch gekennzeichnet, dass** es zwei Prothesen umfasst, von denen jede so ausgebildet ist, dass sie eine Schnittstelle mit einer der unteren Gliedmaßen des Patienten bildet.

7. Vorrichtung nach einem der Ansprüche 5 oder 6, **dadurch gekennzeichnet, dass** das erste und zweite Segment aus Elementen von einstellbarer Länge gebildet sind, so dass ihre Länge an die Gestalt des Patienten angepasst werden kann.

8. Vorrichtung nach einem der vorhergehenden Ansprüche, **dadurch gekennzeichnet, dass** jedes der Gelenke von jeder der Prothesen mit einem elektrischen Servomotor fest verbunden ist, der an ein Untersetzungsgetriebe gekoppelt ist.

9. Vorrichtung nach Anspruch 8, **dadurch gekennzeichnet, dass** die Achse jedes Motors parallel zum benachbarten Prothesensegment ist und dass er die senkrechte Achse des Gelenks mit Hilfe eines Kegelräderpaares antreibt.

10. Vorrichtung nach Anspruch 8, **dadurch gekennzeichnet, dass** der Motor an einem der beiden Prothesensegmente befestigt ist, die mit dem Gelenk verbunden sind, und eine Schraube ohne Ende in eine Drehbewegung versetzt, an der eine Mutter befestigt ist, die ihrerseits eine Kurbel in Bewegung versetzt, die mit dem zweiten Prothesensegment fest verbunden ist und deren Drehachse der Drehachse des Gelenks entspricht.

11. Vorrichtung nach Anspruch 10, **dadurch gekennzeichnet, dass** die Achse jedes Motors senkrecht zum benachbarten Prothesensegment ist.

12. Vorrichtung nach einem der Ansprüche 8 bis 11, **dadurch gekennzeichnet, dass** jeder Motor einen digitalen Positionscodierer umfasst und dass Kraft- und Drehmomentsensoren in jedes Gelenk integriert sind.

13. Vorrichtung nach einem der Ansprüche 8 bis 12, **dadurch gekennzeichnet, dass** die Motoren durch die Steuervorrichtung (31) geregelt werden und dass die Letztgenannte getrennte Steuermittel für jeden der Motoren umfasst.

14. Vorrichtung nach einem der vorhergehenden Ansprüche, **dadurch gekennzeichnet, dass** die orthopädische Vorrichtung an einem horizontalen Rahmen (10) befestigt ist, der so ausgebildet ist, dass er den Rücken und das Becken des Patienten für ein Training seiner unteren Gliedmaßen in horizontaler Position stützt.

15. Vorrichtung nach einem der Ansprüche 1 bis 13, **dadurch gekennzeichnet, dass** die orthopädische Vorrichtung an einem mechanischen Beckengürtel (51) befestigt ist, der so ausgebildet ist, dass er für ein Training der unteren Gliedmaßen des Patienten in vertikaler Position um die Hüften des Patienten angeordnet werden kann, wobei der Beckengürtel Organe zu seiner Befestigung an einer beweglichen Stützvorrichtung umfasst, so dass es dem Patienten möglich ist, sich zu fortzubewegen.

16. Vorrichtung nach Anspruch 15, **dadurch gekennzeichnet, dass** der Beckengürtel an der beweglichen Stützvorrichtung durch einen Gelenkmechanismus befestigt ist.

17. Vorrichtung nach Anspruch 16, **dadurch gekennzeichnet, dass** der Gelenkmechanismus mit einer Motorisierungsvorrichtung versehen ist, die in vertikaler Ebene wirkt und durch Kraft-, Drehmoment- und Positionssensoren geregelt wird, so dass das Gewicht des Patienten während seiner Fortbewegung ganz oder teilweise getragen wird.

18. Vorrichtung nach einem der Ansprüche 15 bis 17, **dadurch gekennzeichnet, dass** die Stützvorrichtung auf Rädern (54, 55, 56) montiert ist.

19. Vorrichtung nach einem der Ansprüche 15 bis 18, **dadurch gekennzeichnet, dass** die Stützvorrichtung ihre eigene Antriebsvorrichtung (65, 66) umfasst, die durch die Steuervorrichtung (31) geregelt wird.

20. Vorrichtung nach einem der Ansprüche 1 bis 19, **dadurch gekennzeichnet, dass** das Steuersystem (31) einen Mikrocomputer umfasst.

21. Vorrichtung nach Anspruch 20, **dadurch gekennzeichnet, dass** das Steuersystem (31) Ablesemittel umfasst, die dazu bestimmt sind, mit tragbaren Datenträgern zusammenzuarbeiten, um die Ausführung von individuellen Trainingsprogrammen zu ermöglichen.

22. Vorrichtung nach einem der vorhergehenden Ansprüche, **dadurch gekennzeichnet, dass** sie Anzeigemittel (40) umfasst, die insbesondere dazu bestimmt sind, den Patienten in Echtzeit über sein Leistungsniveau zu informieren.
